(19) Europäisches Patentamt
European Patent Office
Office européen des brevets

(11) EP 2 399 926 A1

(12) **EUROPEAN PATENT APPLICATION**

(43) Date of publication:
**28.12.2011 Bulletin 2011/52**

(21) Application number: **10195232.3**

(22) Date of filing: **15.12.2010**

(51) Int Cl.:
***C07K 7/02*** *(2006.01)* ***C07K 7/56*** *(2006.01)*
***A61P 25/28*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.06.2010 EP 10166945**

(71) Applicant: **Justus-Liebig-Universität Giessen**
**35390 Giessen (DE)**

(72) Inventors:
- **Schreiner, Peter R.**
  **35435 Wettenberg (DE)**
- **Wanka, Lukas**
  **61206 Wöllstadt (DE)**

(74) Representative: **Stumpf, Peter**
**Kerkrader Straße 3**
**35394 Gießen (DE)**

(54) **Peptides incorporating 3-aminoadamantane carboxylic acids enhance synaptic plasticity and act as neurogenic agents**

(57) The disclosed invention relates to peptides incorporating 3-aminoadamantane-1-carboxylic acids. These peptides are disclosed as surprisingly possessing neurogenic and neurotrophic properties. These pharmacological activities can advantageously be utilized for restoring or maintaining neuronal function in the CNS. In particular, the compounds can be used for treatment and prophylaxis of neurodegenerative diseases, e. g., Alzheimer's disease, Parkinson's disease, Huntington's disease, amyotrophic lateral sclerosis, as well as in post-acute traumatic brain injury, or cerebral ischemia.



Printed by Jouve, 75001 PARIS (FR)

## Description

The disclosed invention relates to peptides incorporating 3-Aminoadamantane-1-carboxylic acids which are disclosed as surprisingly possessing neurogenic and neurotrophic properties. These pharmacological activities can advantageously be utilized for restoring or maintaining neuronal function in the CNS. In particular, the compounds can be used for treatment and prophylaxis of neurodegenerative diseases, e. g., Alzheimer's disease, Parkinson's disease, Huntington's disease, amyotrophic lateral sclerosis, as well as in the treatment of post-acute traumatic brain injury, or cerebral ischemia.

The present invention provides a novel concept of making available peptides, in particular such peptides that resemble functional parts or subsequences of trophic factors, e. g., brain-derived neurotrophic factor (BDNF), ciliary neurotrophic factor (CNTF), nerve growth factor (NGF), neurotrophin-3, glial cell line-derived neurotrophic factor (GDNF), insulin-like growth factor (IGF) or other trophic factors combined with lipophilic 3-aminoadamantane-1-carboxylic acids to enhance potencies and metabolic stabilities of the resulting compounds. While peptides resembling parts or subsequences of the abovementioned trophic factors have been reported in the literature, such peptides also incorporating adamantane-based amino acids, in particular, 3-aminoadamantane-1-carboxylic acids, as building blocks have not been reported as small-molecule analogues of trophic factors with improved pharmacological profile before. These novel peptides modified with 3-aminoadamantane-1-carboxylic acids possess neurogenic and neurotrophic properties and they enhance synaptic plasticity. The preparation of representative examples of this class of compounds is described and their neurogenic and neurotrophic properties are described. Cyclic peptides, for example of the type of neurotrophic peptides presented in this invention (examples 3 and 4) that incorporate two, or more 3-aminoadamantane carboxylic acids as building blocks represent a novel structural motif.

## State of art

Several peptides incorporating 3-aminoadamantane-1-carboxylic acids to be used in medicinal applications were reported in the literature.

Reports of neurogenic or neurotrophic properties of these peptides have not been disclosed previously.

Small peptides incorporating 3-aminoadamantane-1-carboxylic acid as an amino acid building block have been described by Mazur (US 1979-99765 A (1979)). These peptides were synthesized via solution phase chemistry using methyl esters of the adamantane-1-carboxylic acid group and mixed anhydride activation, respectively. These peptides were screened as analgesics. Schreiner et al. (WO 2006010362 A1; L. Wanka, C. Cabrele, M. Vanejews, P. R. Schreiner, Eur. J. Org. Chem. 2007, 1474; C. E. Müller, L. Wanka, K. Jewell, P. R. Schreiner, Angew. Chem. Int. Ed. Engl. 2008, 47, 6180; C. E. Müller, D. Zell, P. R. Schreiner, Chem. Eur. J. 2009, 15, 9647.) described in detail the solution-phase as well as solid-phase synthesis of such peptides using Fmoc-/OtBu methodology. Libraries of peptides incorporating 3-aminoadamantane-1-carboxylic acids are described there for a number of applications in medicinal chemistry, in particular as blockers for GABA transporters and anti-Influenza A compounds, and as catalysts in stereoselective synthesis, respectively. The cytotoxic effect of enkephalin-derived peptides incorporating 3-aminoadamantane-1-carboxylic acid is described by Horvat et al. (S. Horvat, K. Mlinaric-Majerski, L. Glavas-Obrovac, A. Jakas, J. Veljkovic, S. Marczi, G. Kragol, M. Roscic, M. Matkovic, A. Milostic-Srb, J. Med. Chem. 2006, 49, 3136; WO 2006097774 A1, 2006.). An application of these peptides as anti-tumor agents is proposed.

Peptides exhibiting neurotrophic properties have been described in the literature as well. Hughes et al. reported on peptides derived from brain-derived neurotrophic factor (BDNF) which displayed neurogenic properties in cell culture (P. D. O'Leary, R. A. Hughes, J. Neurochem. 1998, 70, 1712; P. D. O'Leary, R. A. Hughes, J. Biol. Chem. 2003, 278, 25738; J. M. Fletcher, R. A. Hughes, J. Pept. Sci. 2006, 12, 515; J. M. Fletcher, C. J. Morton, R. A. Zwar, S. S. Murray, P. D. O'Leary, R. A. Hughes, J. Biol. Chem. 2008, 283, 33375; J. M. Fletcher, R. A. Hughes, Bioorg. Med. Chem. 2009, 17, 2695.) Neuroactive peptides derived from other trophic factors and neuropeptides have also been reported before (see for instance H. H. Chen, Y. C. Tung, B. Li, K. Iqbal, I. Grundke-Iqbal, Neurobiol. Aging 2007, 28, 1148; Y. Matsuoka, Y. Jouroukhin, A. J. Gray, L. Ma, C. Hirata-Fukae, H. F. Li, L. Feng, L. Lecanu, B. R. Walker, E. Planel, O. Arancio, I. Gozes, P. S. Aisen, J Pharmacol Exp Ther 2008, 325, 146.)

The present invention provides a series of peptides and cyclopeptides incorporating 3-aminoadamantane-1-carboxylic acids as building blocks (scheme 1) that display neurotrophic properties.

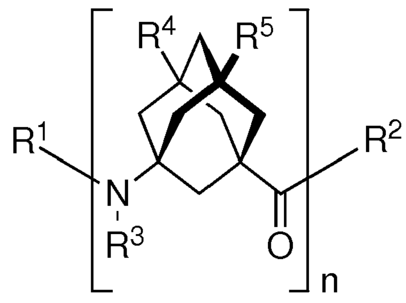

**Scheme 1. Peptidic structures incorporating γ-aminoadamantane-1-carboxylic acids**

These peptides can contain, independently from one another, as $R^1$ and $R^2$, peptide fragments derived from growth factors or neuropeptides as well as amino acid residues to influence pharmacodynamics and pharmacokinetics (e. g., tissue distribution, stability towards proteolytic degradation, permeability through the blood-brain-barrier, etc.), and unnatural α-, β-, γ- and δ-amino acids, preferably 3-aminoadamantane-1-carboxylic acids, or oligopeptides thereof. n can be any number between 1 and 100, preferably between 1 and 10, even more preferably between 1 and 5. In particular, compounds with n = 1, 2, or 3 are preferred.
R $^1$represents a linear or cyclic chain of amino acids incorporating one to 1000, preferably one to 100, even more preferably one to 10 α-, β-, γ- or δ-amino acids in their L- or D-configurations, respectively. In particular, chains of one, two, three, four or five amino acids are preferred.
$R^2$ represents

1. a linear or cyclic chain of amino acids incorporating one to 1000, preferably one to 100, even more preferably one to 10 α-, β-, γ- or δ-amino acids in their L- or D-configurations, respectively. In particular, chains of one, two, three, four or five amino acids are preferred.
or
2. H, $NH_2$, any Halogen, any alkyl or cycloalkyl group with one to 25 carbon atoms, including (but not limited to) methyl, ethyl, propyl, isopropyl, butyl, isobutyl, t-butyl, pentyl, hexyl, heptyl, octyl; any aromatic hydrocarbon with six to ten carbon atoms, e. g., phenyl or naphthyl; any heterocycle (that is, any 5- or 6-membered heterocyclic monocyclic group), e. g., oxazol-2-yl, oxazol-4-yl, 1-imidazolyl, 2-imidazolyl, 4-imidazolyl, 1-pyrrolyl, 2-pyrrolyl, 2-pyridyl, 3-pyridyl, 4-pyridyl, 1-piperidyl, 2-piperidyl, 3-piperidyl, 4-piperidyl

In one embodiment, the peptide chains $R^1$ and $R^2$ are connected to one another, resulting in a cyclic peptide that incorporates one or more 3-aminoadamantane-1-carboxylic acids as well as 1 to 100, preferably 1 to 10, even more preferably 2 to 5, α-, β-, γ-, or δ-amino acids in their L- or D-configurations, respectively. In particular, cyclopeptides with a total number of 4 to 10 amino acids are preferred.
In another embodiment, $R^1$ and $R^2$ are not connected to one another, resulting in in a linear peptide that incorporates one or more 3-aminoadamantane-1-carboxylic acids as well as 1 to 100, preferably 1 to 10, even more preferably 2 to 5, α-, β-, γ-, or δ-amino acids in their L- or D-configurations, respectively. In particular, cyclopeptides with a total number of 4 to 10 amino acids are preferred.

In the most preferred embodiment, $R^1$ represents a linear or cyclic chain of amino acids as defined above, and $R^2$ represents an amino group ($NH_2$). Most suitably, $R^1$ represents an amino acid chain of one, two, three, four or five amino acids, and $R^2$ represents $NH_2$.

$R^3$ can be H, methoxy, any alkyl or cycloalkyl group with one to 25 carbon atoms.
Additionally, $R^4$ and $R^5$ also can be, independently from one another, H, $NH_2$, any Halogen, any alkyl or cycloalkyl group with one to 25 carbon atoms, including (but not limited to) methyl, ethyl, propyl, isopropyl, butyl, isobutyl, t-butyl, pentyl, hexyl, heptyl, octyl; any aromatic hydrocarbon with six to ten carbon atoms, e. g., phenyl or naphthyl; any heterocycle (that is, any 5- or 6-membered heterocyclic monocyclic group), e. g., oxazol-2-yl, oxazol-4-yl, 1-imidazolyl, 2-imidazolyl, 4-imidazolyl, 1-pyrrolyl, 2-pyrrolyl, 2-pyridyl, 3-pyridyl, 4-pyridyl, 1-piperidyl, 2-piperidyl, 3-piperidyl, 4-piperidyl.

The peptides are conveniently synthesized by solid phase peptide synthesis methods; however, synthesis in solution is also feasible.

Several of these peptides exhibit neurogenic and neurotrophic properties and enhance synaptic plasticity in cell culture as well as *in vivo* in mice. The following examples are given to illustrate the invention. It is not limited to them in any manner.

## Problem

The problem of the underlying invention is to foresee a) a new class of chemical substances and b) a known class of chemical substances for the manufacture of a new medicament for the treatment of neurodegenerative diseases including, but not limited to, Parkinson's disease, Alzheimer's disease, Huntington's disease, and amyotrophic lateral sclerosis, as well as post-acute traumatic brain injury or cerebral ischemia.

## Solution

Surprisingly it was found, that peptides incorporating 3-aminoadamantane-1-carboxylic acids and further amino acids, in particular proteinogenic $\alpha$-amino acids in L- or D-configurations and unnatural amino acids as described in claim 1 and the known substances as described in claim 2 possess the desired properties.

## Examples

## Examples

### (a) General remarks:

All peptides were synthesized by standard solid phase peptide synthesis (SPPS) methods following the Fmoc-strategy. 3-(9-Flourenylmethoxycarbonyl)aminoadamantane-1-carboxylic acid (Fmoc-$^{A}$Gly) was synthesized as described previously. Fmoc-$\alpha$-amino acids, activation reagents and other chemicals used were purchased from Novabiochem and used as supplied. Solvents used were peptide synthesis grade. Automated SPPS was performed on Rink amide AM resin (200 - 400 mesh, Novabiochem) using a Protein Technologies PS-3 automated peptide synthesizer. Loading (2 x 40 min) as well as chain elongation (2 x 20 min) were performed via double-couplings using 2 x 4 equivalents (over resin substitution) of the respective Fmoc-amino acid, 2-(1H-Benzotriazole-1-yl)-1,1,3,3-tetramethylaminium hexafluorophosphate (HBTU), 1-Hydroxybenzotriazole hydrate (HOBt), and excess *N,N*-diisopropylethlamine (DIPEA) in DMF. Acetylation was performed using 10 equivalents of acetic anhydride and DIPEA over resin loading in DMF for 20 min. Adamantanoylation was performed using adamantane-1-carboxylic acid (Acros Organics) in a double-coupling procedure with HBTU/HOBt activation as described above for the chain elongation steps. Removal of the temporary Fmoc-protective group was performed using 20% piperidine in DMF for 2 x 20 min or 20% piperidine and 1% DBU in DMF for 3 x 3 min. After each chain elongation and Fmoc-cleavage step, the resin was washed with DMF (6 x 30 s). The peptides were cleaved from the resin with trifluoroacetic acid (TFA) / water / triisopropylsilane (95 : 2.5 : 2.5) for 3 h and precipitated by the addition of ice-cold diethyl ether. The peptides were collected by centrifugation. The crude precipitates were resuspended in fresh ice-cold diethyl ether and centrifuged another two times. After dissolving in water /acetic acid (2 : 1) and extraction with diethyl ether / hexanes (1 : 1), the aqueous solution of the crude peptides was lyophilized. The peptides were purified by semipreparative reversed-phase HPLC using a Restek RP18 column (21.2 x 250 mm, 5 $\mu$m, 300 Å) using gradients of solvent B in solvent A (A: water, 0.1 % TFA; B: acetonitril, 0.1 % TFA) at 3.5 mL/min flow rate. Product fractions were analyzed by analytical HPLC using an Restek C8 column (4.6 x 150 mm), also using linear gradients of solvent B in solvent A at 1 mL/min flow. Both analytical and semipreparative HPLC were monitored at 215 nm using a variable wavelength detector. Product fractions containing the peptides in >95% purity as analyzed by analytical HPLC were pooled, lyophilized and used for the present study. Proton NMR were recorded on a Bruker 400 MHz spectrometer or a Varian 600 MHz instrument. Proton chemical shifts are reported in ppm ($\delta$) relative to internal tetramethylsilane (TMS, $\delta$ 0.0 ppm) or the solvent residual peaks.
Data are reported as follows: chemical shift (multiplicity [singlet (s), doublet (d), triplet (t), quartet (q), multiplet (m)], coupling constants [Hz], integration). The spectra were obtained at 25 °C. ESI-MS spectra were recorded on an Finnigan LCQ duo mass spectrometer.
All the following peptide syntheses can also be performed using different additives, stoichiometries, solvents, and coupling times as well as various temperatures. Furthermore, microwave-assisted peptide synthesis is also possible. The following examples and the general procedures above, therefore, only give an estimate of suitable synthesis conditions but do not limit the synthesis of the respective compounds to the protocols described below.

**Example 1: Ac-Asp-Gly-Gly-Leu-$^{A}$Gly-$NH_2$ ("Peptide 021)":**

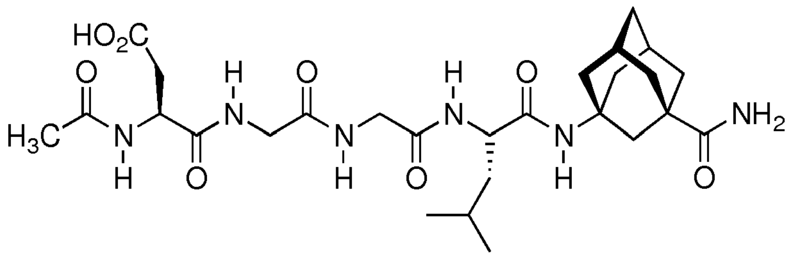

Preparative HPLC: 15 - 45% solvent B in solvent A in 80 min., product fractions eluted at 40 - 43 min. Analytical HPLC: 10 - 60% solvent B in solvent A in 20 min., rt = 9.5 min.

**$^1$H-NMR** (600 MHz, [$D_6$]DMSO): δ = 0.83 (d, *J*= 6.5 Hz, 3 H, Leu-*H*δ); 0.87 (d, *J*= 6.6 Hz, 3 H, Leu-*H*δ); 1.36 - 1.47 (m, 2 H, Leu-*H*β); 1.49 - 1.59 (m, 3 H, Leu-*H*γ and adamantane-C$H_2$); 1.62 - 1.71 (m, 4 H, 2 × adamantane-C$H_2$); 1.79 - 1.90 (m, 4 H, 2 × adamantane-C$H_2$); 1.86 (s, 3 H, acetyl-C$H_3$); 1.92 (br. s, 2 H, adamantane-C$H_2$); 2.06 - 2.12 (m, 2 H, 2 × adamantane-C*H*); 2.70 (dd, *J* = 16.6 and 5.4 Hz, 1 H, Asp-*H*β); 3.66 - 3.75 (m, 5 H, 4 × Gly-*H*α and Asp-*H*β); 4.21 - 4.27 (m, 1 H, Leu-*H*α); 4.51 - 4.57 (m, 1 H, Asp-*H*α); 6.71 (s, 1 H, CON$H_2$); 6.98 (s, 1 H, CON$H_2$); 7.36 (br. s, 1 H, $^{A}$Gly-N*H*); 7.69 (d, *J* = 8.4 Hz, 1 H, Leu-N*H*); 7.99 (t, *J* = 5.9 Hz, 1 H, Gly-N*H*); 8.20 (t, *J* = 5.7 Hz, 1 H, Gly-N*H*); 8.24 (t, *J* = 7.6 Hz, 1 H, Asp-N*H*); 12.32 (br. s, 1 H, Asp-$CO_2H$). **MS** (ESI): m/z = 579.4 $[M + H]^+$ (calcd. 579.3).

**Example 2: 1-AdCO-DGGL$^{A}$G-$NH_2$ ("Peptide 022"):**

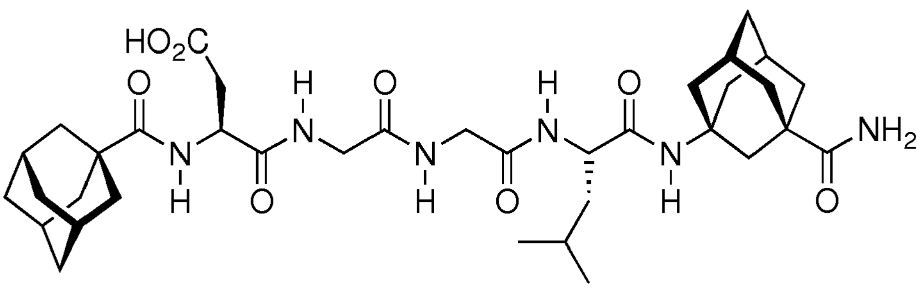

Preparative HPLC: 10 - 43% solvent B in solvent A in 79 min, then 43 - 60% solvent B in solvent A in 10 min., then 20 min. at 60% solvent B in solvent A. Product fractions eluted at 83 - 86 min. Analytical HPLC: 10 - 60% B in A in 20 min., rt = 15.5 min.

**$^1$H-NMR** (600 MHz, [$D_6$]DMSO): δ = 0.82 (d, *J*= 6.8 Hz, 3 H, Leu-*H*δ); 0.87 (d, *J*= 6.8 Hz, 3 H, Leu-*H*δ); 1.35 - 1.45 (m, 2 H, Leu-*H*β); 1.49 - 1.59 (m, 3 H, Leu-*H*γ and adamantane-C$H_2$); 1.61 - 1.70 (m, 10 H, 5 × adamantane-C$H_2$); 1.75 - 1.80 (m, 6 H, 3 × adamantane-C$H_2$); 1.80 - 1.89 (m, 4 H, 2 × adamantane-C$H_2$); 1.92 (br. s, 2 H, adamantane-C$H_2$); 1.94 - 1.98 (m, 3 H, 3 × adamantane-C*H*); 2.04 - 2.11 (m, 2 H, 2 × adamantane-C*H*); 2.56 (dd, *J* = 16.0 and 8.0 Hz, 1 H, Asp-*H*β); 2.74 (dd, *J* = 16.1 and 5.5 Hz, 1 H, Asp-*H*β); 3.66 - 3.75 (m, 4 H, 4 × Gly-*H*α); 4.21 - 4.27 (m, 1 H, Leu-*H*α); 4.53 - 4.58 (m, 1 H, Asp-*H*α); 6.71 (s, 1 H, CON$H_2$); 6.98 (s, 1 H, CON$H_2$); 7.35 (s, 1 H, $^{A}$Gly-N*H*; 7.62 (d, *J* = 7.7 Hz, 1 H, Leu-N*H*); 7.71 (d, *J* = 8.5 Hz, 1 H, Asp-N*H*); 7.88 (t, *J* = 5.5 Hz, 1 H, Gly-N*H*); 8.03 (t, *J* = 5.9 Hz, 1 H, Gly-N*H*); 12.20 (br. s, 1 H, Asp-$CO_2H$). **MS** (ESI): m/z = 699.4 $[M + H]^+$ (calcd. 699.4).

**Example 3: cyclo-[L-Asp-D-Lys-$^{A}$Gly-L-Asp-D-Lys-$^{A}$Gly] ("Peptide 140")**

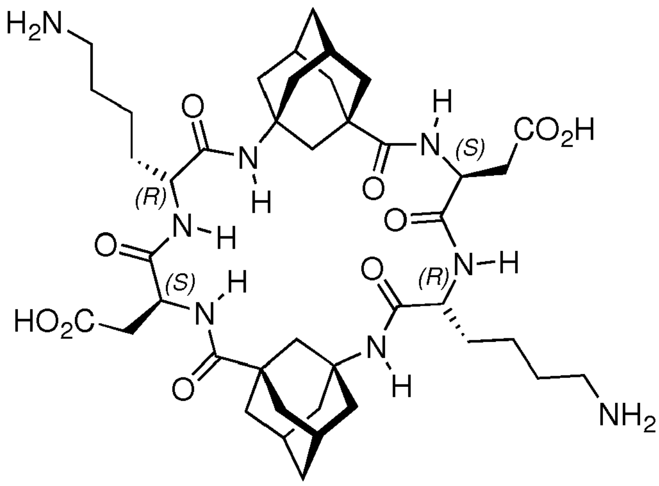

Commercially available Wang resin (100-200 mesh) was loaded with Fmoc-Asp-ODmab to a substitution level of 0.40 mmol/g. Endcapping of nonfunctionalized hydroxy groups was subsequently performed using the acetylation protocol described above (10 eq. DIPEA and acetic anhydride in DMF for 20 min). Peptide synthesis of the linear precursor was performed as described above using double-coupling procedures. After cleavage of the final Fmoc-protective group, the C-terminal Dmab protective group was cleaved by shaking with 2% hydrazine hydrate in DMF (4 x 5 min). After six times washing with DMF, cyclization was initiated using 5 equivalents PyBOP and HOBt, as well as 10 equivalents DIPEA (3 x 16 h). After washing and drying of the resin-bound cyclopeptide, cleavage and workup of the crude peptide was performed similar to the protocols given above for the linear example peptides.
Preparative HPLC: 10 - 60% solvent B in solvent A in 120 min. Product fractions eluted at 45.4 - 48.9 min. Analytical HPLC: 10 - 60% B in A in 20 min., rt = 7.60 min.
**$^1$H-NMR** (400 MHz, [$D_6$]DMSO): $\delta$ = 1.20 - 1.40 (m, 4 H, Lys-*H*$\gamma$); 1.40 - 1.69 (m, 20 H, adamantane-C*H* and adamantane-C$H_2$); 1.69 - 1.80 (m, 4 H, Lys-*H*$\delta$); 1.89 - 2.03 (m, 2 H, Lys-*H*$\beta$); 2.03 - 2.12 (m, 4 H, adamantane-C$H_2$); 2.12 - 2.27 (m, 4 H, adamantane-C*H*); 2.30 - 2.44 (m, 2 H, Lys-*H*$\beta$); 2.53 - 2.67 (m, 4 H, Asp-*H*$\beta$); 2.68 - 2.81 (m, 4 H, Lys-*H*$\varepsilon$); 3.82 - 3.92 (m, 2 H, Lys-*H*$\alpha$); 4.35 (q, J = 6.8 Hz, 2 H, Asp-*H*$\alpha$); 6.93 (s, 2 H, $^A$Gly-N*H*); 7.71 (br. s, 4 H, Lys-N$H_2$($\varepsilon$)); 7.80 (d, J = 6.0 Hz, 2 H, Asp-N*H*); 8.30 (d, 2 H, J = 8.0 Hz, Lys-N*H*); 12.30 (br. s, 2 H, Asp-CO$_2$*H*). **MS** (ESI): m/z = 841.6 [M + H]$^+$ (calcd. 841.5).

**Example 4: cyclo-[Asp-Cit-$^A$Gly-Cit-Cit-$^A$Gly] ("Peptide 144")**

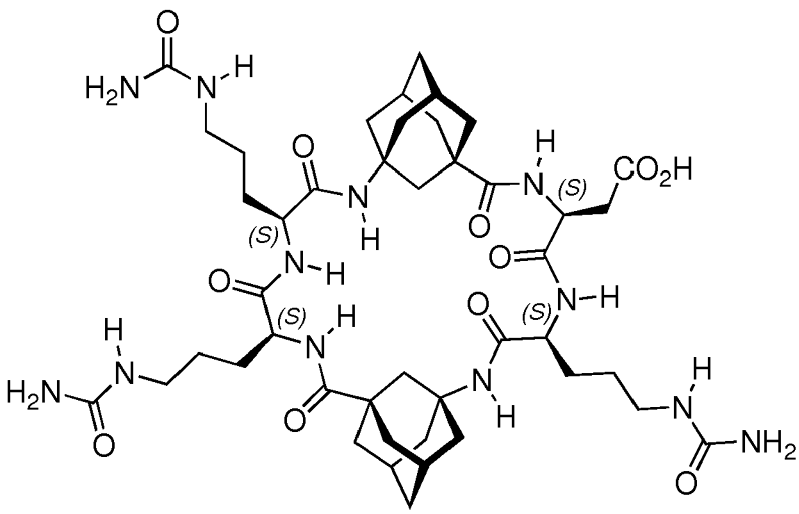

This cyclic peptide was prepared in the same fashion as is described in example 3.
Preparative HPLC: 10 - 40% solvent B in solvent A in 90 min. Product fractions eluted at 51.3 - 52.3 min. Analytical HPLC: 10 - 60% B in A in 20 min., rt = 7.35 min.
**$^1$H-NMR** (400 MHz, [$D_6$]DMSO): δ = 1.20 - 1.50 (m, 6 H, Cit-*H*γ); 1.51 - 1.82 (m, 24 H, adamantane-C*$H_2$*); 1.82 - 2.03 (m, 6 H, Cit-*H*β); 2.03 - 2.23 (m, 4 H, adamantane-C*H*); 2.36 (t, J = 11.0 Hz, 1 H, Cit-*H*β); 2.43 - 2.56 (m, 1 H, Asp-*H*β); 2.69 (dd, $J_1$ = 10 Hz, $J_2$ = 5 Hz, 1 H, Asp-*H*β); 2.92 (t, J = 6.5 Hz, 5 H, Cit-*H*δ); 3.91 (q, J = 7.0 Hz, 1 H, Cit-*H*α); 4.03 (q, J = 7.2 Hz, 1 H, Cit-*H*α); 4.09 (q, J = 7.2 Hz, 1 H, Cit-*H*α); 4.50 (q, J = 7.1 Hz, 1 H, Asp-*H*α); 5.99 (br. s, 3 H, Cit-N*H* (ε)); 7.16 (s, 2 H, $^A$Gly-N*H*); 7.28 (s, 1 H, Cit-N*H*); 7.30 (s, 1 H, Asp-N*H*); 7.34 (s, 2 H, Cit-N*H*); 7.64 - 7.74 (m, 6 H, Cit-N*$H_2$*). **MS** (ESI): m/z = 941.7 $[M + H]^+$ (calcd. 941.5).

**In vivo and in vitro screens**

***General remarks:***

All *in vivo* studies for characterization of peptides (stereology and behavioral analysis) were performed on 8-10-month-old female retired breeders of C57Bl6 background. Animals were acclimatized for at least 3 weeks to exclude occasional pregnant mice from the studies. Mice were group-housed (3 animals per cage) with a 12 : 12 hours light / dark cycle and with free access to food and water. All procedures were conducted in accordance with approved protocols from our institutional Animal Welfare Committee. For the peptide treatment, mice (8 animals/group) received subcutaneous implants of long term continuous release depot pellets containing Peptide021 or Peptide022 at 25 nmol/day for 35 days of continuous dosing (Innovative Research of America, Sarasota, FL). For the control group (8 animals), pellets consisted of the carrier polymer only. Mice were anesthetized with 2.5% Avertin (0.38 ml for a 25g animal). Under sterile conditions, pellets were implanted subcutaneously along the anterolateral aspect of the right shoulder with a precision trochar (Innovative Research of America, Sarasota, FL). After recovery of anesthesia, animals were transferred to the animal colony. No complications associated with the implantation and treatment were observed. To investigate neurogenesis, BrdU (Sigma, St. Louis, MO) was given as two daily i.p. injections (100 mg/kg/dose) for five days starting on day 2 of peptides treatment.

**General examination**

The physical state and condition of animals were carefully monitored throughout the treatment by evaluating grooming, physical state and clasping reflex. Body weight was also recorded.

**One-trial object recognition test**

In the one-trial object recognition task, animals are exposed to two different objects which they have to identify as novel or familiar based on the memory of an earlier experience with one of the two objects they encountered in the same open field. The familiar object is explored shorter than the novel encountered one because the representation of the former is still available in memory. The one-trial object recognition task tests some aspects of episodic memory but is limited to memory of an object (what), the location of an object (where), and the context in which is was encountered (which). However, the temporal dimension of the episode remains inaccessible to the experimentator, and because of this reason this task in animals is considered a test of short term memory (Ennaceur, 2010). The test used was an adaptation of the procedure previously described by Sargolini and collaborators (2003). The testing apparatus was a classic open field (i.e. a PVC square arena, 50 x 50 cm, with walls 40 cm high). The open field was placed in a part of the room separated from the experimentator with a black opaque curtain. The open field was surmounted by a video camera connected to a computer. Three objects were employed in this task. The general procedure consisted of three different phases: a familiarization phase (one session of 20 min), a sample phase, and a test phase. On the first day, mice were individually submitted to the familiarization session during which they were introduced in the empty arena in order to become familiar with the apparatus. This familiarization session allowed to record a baseline level of locomotor activity (measuring the distance covered in the open field) and of anxiety (measuring the time the animals spent in the centre of the arena during the first 5 minutes). On the second day each mouse was first submitted to the sample phase (session 1, 10 min) for which two identical objects were placed in a symmetric position from the centre of the arena. After a 15-min delay during which the mouse returned to its home cage, it was reintroduced in the arena to perform the test phase (session 2, 10 min). The mouse was then exposed to two objects: a familiar object (previously presented during the sample phase) and a new object, placed at the same location as during the sample phase. Data collection was performed using a videotracking system (Smart version 2.0.14 software, Pan Lab/San Diego Instruments). Object discrimination ($O_D$) during the test phase was evaluated as

$$O_D = \frac{\text{(time spent close to new object)}}{\text{(time spent close to new object)} + \text{(time spent close to old object)}} \cdot 100$$

**Spatial reference memory task in the water maze**

Spatial reference learning and memory were evaluated in the water maze using a procedure adapted from that previously described by Morris et al. (1982). The test required that mice used a spatial navigational strategy based on a spatial representation of the environment to find a fixed submerged escape platform. The procedure was performed in a 180 cm diameter circular tank. The pool was filled with water (21±1) °C made opaque by adding white non-toxic paint. Acquisition started with the escape platform (15 cm diameter submerged 1 cm below water surface) in the Northwest quadrant and each animal was given 90 s to find the platform. If the mouse did not find the platform in 90 s, it was gently guided to it. At the end of each trial, the mouse was left on the platform for 20 s, then dried and returned to its home cage until the next trial. Five such acquisition trials were given on each day for four consecutive days. A test for retention, or probe trial (PT), was given 24 hours later. During the probe trial the mouse was allowed to swim in the tank without the escape platform for 60 seconds. This was followed by second and third probe trials 15 and 30 days after the first probe trial. The measures of learning were the time and distance covered to reach the escape platform. For the probe trial, the tank was divided into four imaginary quadrants and a small zone where the escape platform had been. The measures of retention were calculated index as the ratio of time spent or distance covered in target quadrant over them in the three other quadrants. Mouse behavior in the water maze was monitored by a Samsung Digital Camera (SDC 4304) mounted to the ceiling and tracked and timed by a SMART (Pan Lab/San Diego Instruments) version 2.0.14 software.

**Immunohistochemistry**

At the end of the behavioral experiment, animals were anesthetized with an overdose of sodium pentobarbital (50 mg/kg) and transcardially perfused with 0.1 M phosphate buffered saline (PBS). After perfusion, the brains were removed from the skull, the left hemisphere was immediately frozen for future biochemical analysis and the right hemisphere was immersion fixed in 4% paraformaldehyde in 0.1 M PBS for at least 24 hours at room temperature. Tissues were equilibrated and stored in 30% sucrose solution at 4 °C until sectioning. The brains were sectioned sagittaly on a freezing sliding microtome at 40 μm through the entire hippocampus and the sections were stored in glycol anti-freeze solution (Ethylene glycol, glycerol and 0.1 M PBS in 3:3:4 ratio) at -20°C until further processing. For double labeling of BrdU and NeuN, brain sections were pretreated with 2 M HCl at 37 °C for 30 min and neutralized with 0.1 M borate buffer (pH 8.5) for 10 minutes. Tissue sections were incubated first for 30 min with blocking buffer (4% normal goat serum + 0.1 % Tween-20 in PBS) and then overnight at 4°C in the presence of BrdU (Millipore Corporation, Billerica, MA) and NeuN (Millipore Corporation) antibodies diluted 1:400 and 1:100, respectively. To determine the integrity of presynaptic terminals, tissues were labeled with anti-synaptophysin (1:200; Clone SY38, Millipore Corporation) or anti-synapsin I (1:200; Stressgen Biotechnologies Corporation, Victoria, B.C., Canada). The brain sections were incubated in primary antibody over night at 4°C, respectively. Alexa 488 and 594 (1:500; Invitrogen, Carlsbad, CA) were used as secondary antibodies. All images were obtained using Nikon Eclipse 90i and D-Eclipse C1 microscopes (Nikon Corporation, Japan). Neurogenesis in the dentate gyrus was evaluated by counting the number of BrdU-positive and BrdU/NeuN-double-positive cells in the dentate gyrus (DG). The number of positive cells was determined in every fifth section in a series of 40 μm sagittal sections throughout the DG using unbiased sterology (West et al., 1991). All BrdU-positive cells in the subgranular zone (SGZ) and granule cell layer (GCL) were counted using a fluorescent microscope (Nikon Eclipse 90i, Nikon Corporation, Japan). Double labeled cells were assessed by a confocal imaging system (D-Eclipse C1, Nikon Corporation). Employing principles of unbiased stereology, the optical fractionator method was used to estimate cell counts for the DG (West et al. 1991). For each brain, at least 100 cells were counted based on coefficient of error determinations. For quantitative analysis of the expression of synaptophysin and synapsin I in DG, every tenth section in a series of 40 μm coronal sections throughout the hippocampus was analyzed. The entire area of GCL was outlined. Maximum projection images were then generated based on confocal z-stacks, and antibody staining was quantified by measuring the mean optical density (OD) with the help of NIH Image J program, version 1.32j (http://rsb.info.nih.gov/ij/).

**Analysis of STAT3 phosphorylation in HepG2 cells**

To investigate the molecular mechanism of action of Peptide021 in LIF signaling, HepG2 human hepatoma cell (ATCC, Manassas, VA) at 80% confluence were treated with different concentrations of Peptide021 and with 0.25

nM LIF (Chemicon International Inc., Temecula, CA) for 15 min. The cells were lysed and subjected to Western blots developed with antibodies anti-phospho-Tyr 705 STAT3 and anti-STAT3 (Cell signaling Technology, Danvers, MA).

**Statistical analysis**

All statistical analyses were performed with STATISTICA 6.0 (StatSoft, Inc. Tulsa, OK). Data are represented as mean ± SEM. ANOVAs with *post hoc* Fisher LSD test were used for data analyses, except data from STAT3 phosphorylation, which were analyzed by Student's t-tests. Differences with $p<0.05$ were considered significant.

**Figure Legends**

**Figure 1: Peptides incorporating $^{A}$Gly improve cognition.**
Peptides 021 and 022 did not induce any effect on body weight (**A**), exploratory activity (**C**) or swim speed (**D**) but reduced anxiety level of mice (**B**). (**E-F**) Peptide 021 significantly improved the ability to discriminate a new object versus a familiar object. *p<0.05; Student t-test. (**G**) Peptides 021 and 022 increased performance in the learning of spatial memory task in water maze. **p<0.001; two-way ANOVA with *post hoc* Fisher LSD test. (**H**) Peptide 021 improved performances in the first probe trial, but treatment with Peptide 021 had no longer effect after 15 days (PT2) or 30 days (PT3) of the end of the preatment with the peptide. *p<0.05; Student t-test.

**Figure 2: Peptide 021 promotes neurogenesis in the DG.**
(**A**) Representative picture of double labeled BrdU (red) and NeuN (green) positive cells. Scale bar represents 20 μm. (**B**) Numbers of BrdU positive cells were significantly increased in Peptide 021 treated animals in the GCL, in the SGZ and in the total DG. (**C**) Peptide 021 treatment significantly increased the number of BrdU/NeuN positive cells in the GCL and in the total DG. * p<0.05, two- way ANOVA and *post hoc* Fisher LSD test.

**Figure 3. Peptide 021 promotes synaptic plasticity in the DG.**
Peptide 021 significantly promoted expression of synaptophysin (**A**) and synapsin I (**B**) in the DG. ***p<0.001, two-way ANOVA and *post hoc* Fisher LSD test. Scale bars represent 100 μm.

**Figure 4. Inhibition of LIF-induced STAT3 phosphorylation by Peptide 021 in a dose-dependent manner in HepG2 cells.**
HepG2 cells were treated with different concentrations of Peptide 021 together with 0.25 nM LIF for 15 min, and then the STAT3 phosphorylation at Tyr705 (pY-STAT3) was determined by Western blots. The pY-STAT3 value was normalized to total STAT3 expression. Data are presented as percentages of the value from cells treated with 0.25 nM LIF alone (100%). *p<0.01.

**Claims**

1. A compound according to formula (I)

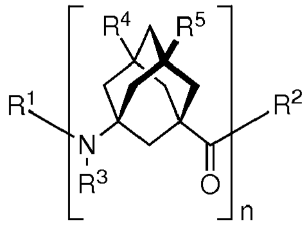

(I),

wherein

$R^1$represents a linear or cyclic chain of amino acids incorporating one to 1000, preferably one to 100, even more preferably one to 10 α-, β-, γ- or δ-amino acids in their L- or D-configurations, respectively,

$R^2$ represents

3. a linear or cyclic chain of amino acids incorporating one to 1000, preferably one to 100, even more preferably one to 10 α-, β-, γ- or δ-amino acids in their L- or D-configurations, respectively. In particular, chains of one, two, three, four or five amino acids are preferred.
or
4. H, $NH_2$, any Halogen, any alkyl or cycloalkyl group with one to 25 carbon atoms, including (but not limited to) methyl, ethyl, propyl, isopropyl, butyl, isobutyl, t-butyl, pentyl, hexyl, heptyl, octyl; any aromatic hydrocarbon with six to ten carbon atoms, e. g., phenyl or naphthyl; any heterocycle (that is, any 5- or 6-membered heterocyclic monocyclic group), e. g., oxazol-2-yl, oxazol-4-yl, 1-imidazolyl, 2-imidazolyl, 4-imidazolyl, 1-pyrrolyl, 2-pyrrolyl, 2-pyridyl, 3-pyridyl, 4-pyridyl, 1-piperidyl, 2-piperidyl, 3-piperidyl, 4-piperidyl

$R^3$ represents H, methoxy, any alkyl or cycloalkyl group with one to 25 carbon atoms,
and
$R^4$ and $R^5$ represent, independently from one another, H, $NH_2$, any Halogen, any alkyl or cycloalkyl group with one to 25 carbon atoms, including (but not limited to) methyl, ethyl, propyl, isopropyl, butyl, isobutyl, t-butyl, pentyl, hexyl, heptyl, octyl; any aromatic hydrocarbon with six to ten carbon atoms, e. g., phenyl or naphthyl; any heterocycle (that is, any 5- or 6-membered heterocyclic monocyclic group), e. g., oxazol-2-yl, oxazol-4-yl, 1-imidazolyl, 2-imidazolyl, 4-imidazolyl, 1-pyrrolyl, 2-pyrrolyl, 2-pyridyl, 3-pyridyl, 4-pyridyl, 1-piperidyl, 2-piperidyl, 3-piperidyl, 4-piperidyl.

**2.** A compound according to claim 1, wherein n represents 1, 2, or 3.

**3.** A compound according to claim 1 or 2, wherein the peptide chains $R^1$ and $R^2$ are connected to one another, resulting in a cyclic peptide that incorporates one or more 3-aminoadamantane-1-carboxylic acids as well as 1 to 100, preferably 1 to 10, even more preferably 2 to 5, α-, β-, γ-, or δ-amino acids in their L- or D-configurations.

**4.** A compound according to claim 3, wherein the wherein the peptide chains $R^1$ and $R^2$ are connected to one another, resulting in a cyclic peptide, and the total number of amino acids is 4 to 10.

**5.** A compound according to claim 1 or 2, wherein $R^1$ and $R^2$ are not connected to one another, resulting in in a linear peptide that incorporates one or more 3-aminoadamantane-1-carboxylic acids as well as 1 to 100, preferably 1 to 10, even more preferably 2 to 5, α-, β-, γ-, or δ-amino acids in their L- or D-configurations.

**6.** A compound according to claim 5, wherein $R^1$ represents a linear or cyclic chain of amino acids as defined above, and $R^2$ represents an amino group ($NH_2$).

**7.** A compound according to claim 6, wherein R1 represents an amino acid chain of one, two, three, four or five amino acids, and $R^2$ represents $NH_2$.

**8.** Use of a compound according to one of the claims 1 to 7 in manufacturing a medicament for the treatment of defects of the central nervous system.

**9.** Use of a compound according to claim 8 in manufacturing a medicament for the treatment of Alzheimer's disease, Parkinson's disease, Huntington's disease, amyotrophic lateral sclerosis, post-acute traumatic brain injury, or cerebral ischemia.

**Fig. 1**

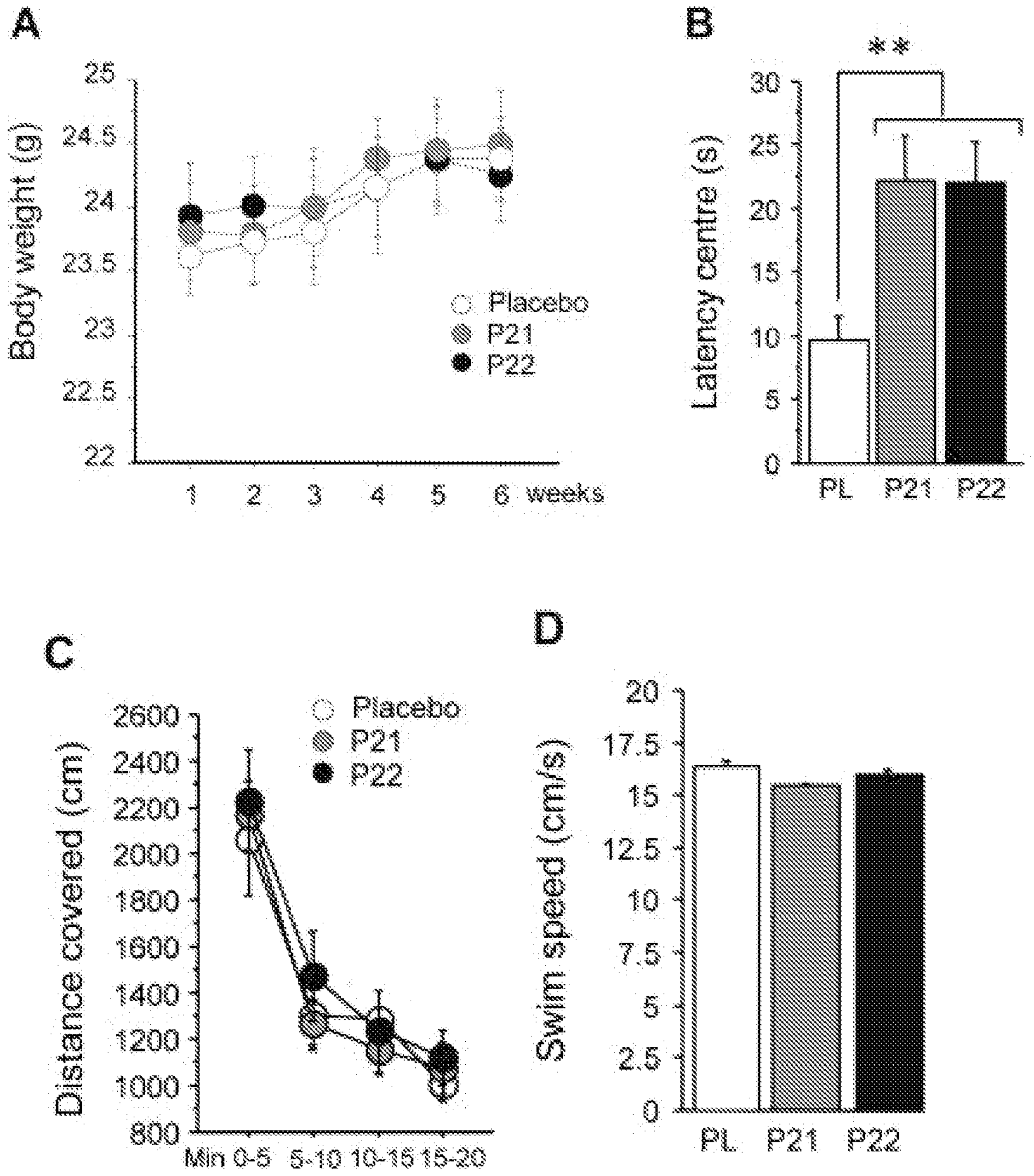
A
Body weight (g)
25
24.5
24
23.5
23
22.5
22
1
2
3
4
5
6 weeks
Placebo
P21
P22
B
**
Latency centre (s)
30
25
20
15
10
5
0
PL
P21
P22
C
Distance covered (cm)
2600
2400
2200
2000
1800
1600
1400
1200
1000
800
Placebo
P21
P22
Min 0-5
5-10
10-15
15-20
D
Swim speed (cm/s)
20
17.5
15
12.5
10
7.5
5
2.5
0
PL
P21
P22

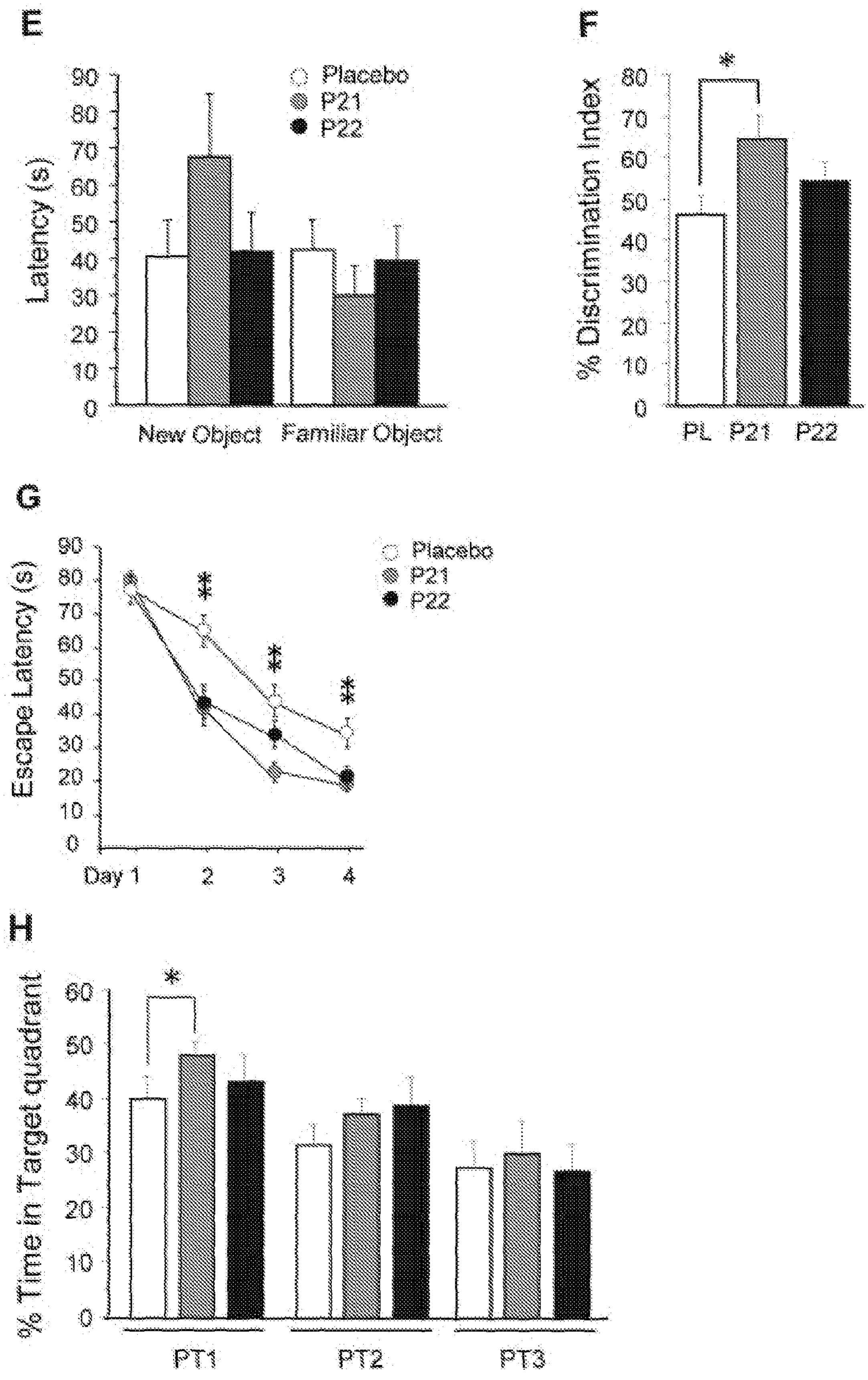
E
Placebo
P21
P22
Latency (s)
0
10
20
30
40
50
60
70
80
90
New Object
Familiar Object
F
*
% Discrimination Index
0
10
20
30
40
50
60
70
80
PL
P21
P22
G
Placebo
P21
P22
Escape Latency (s)
0
10
20
30
40
50
60
70
80
90
**
**
**
Day 1
2
3
4
H
*
% Time in Target quadrant
0
10
20
30
40
50
60
PT1
PT2
PT3

Fig. 2

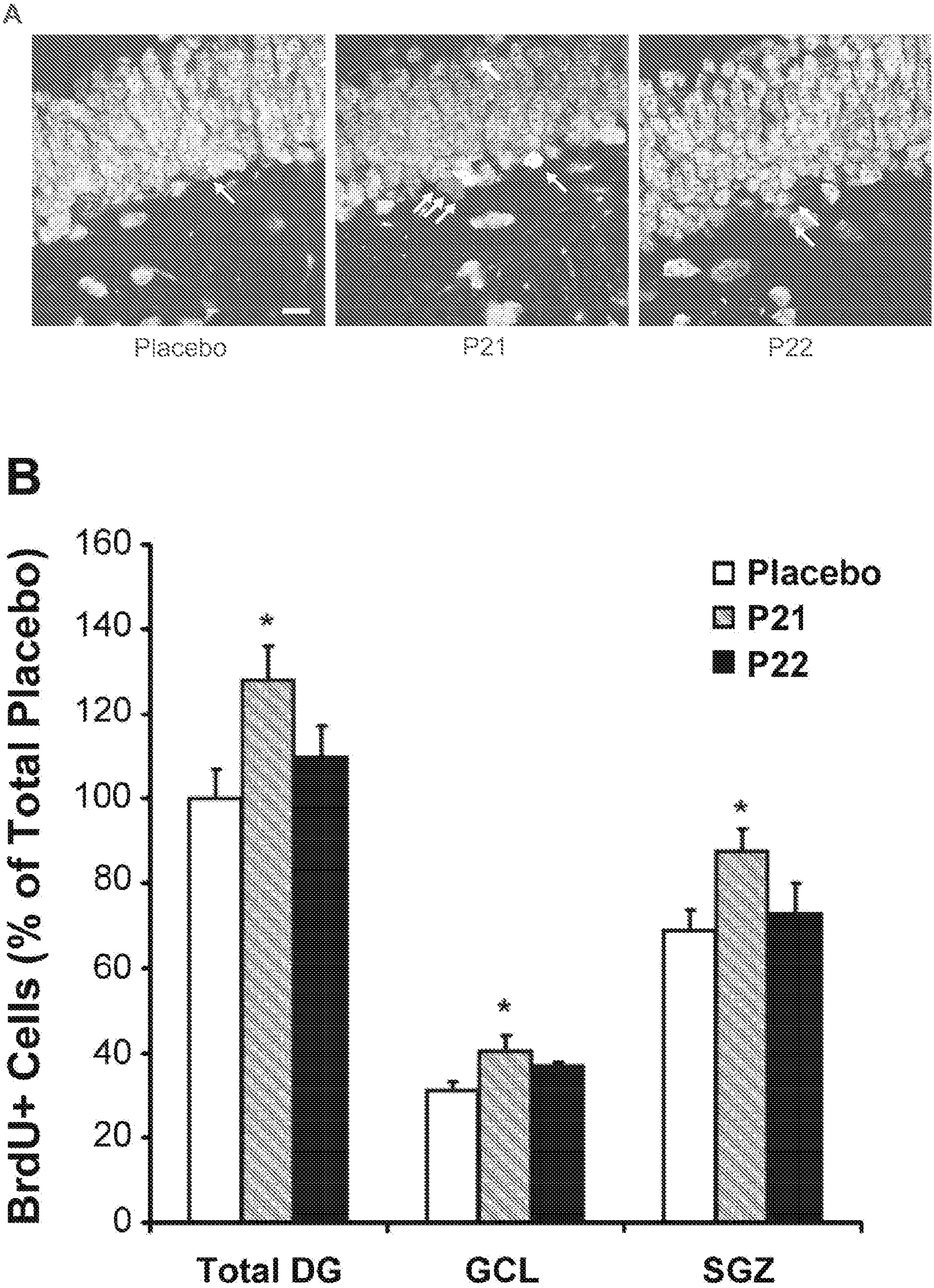
A
Placebo
P21
P22
B
BrdU+ Cells (% of Total Placebo)
160
140
120
100
80
60
40
20
0
Placebo
P21
P22
*
*
*
Total DG
GCL
SGZ

C

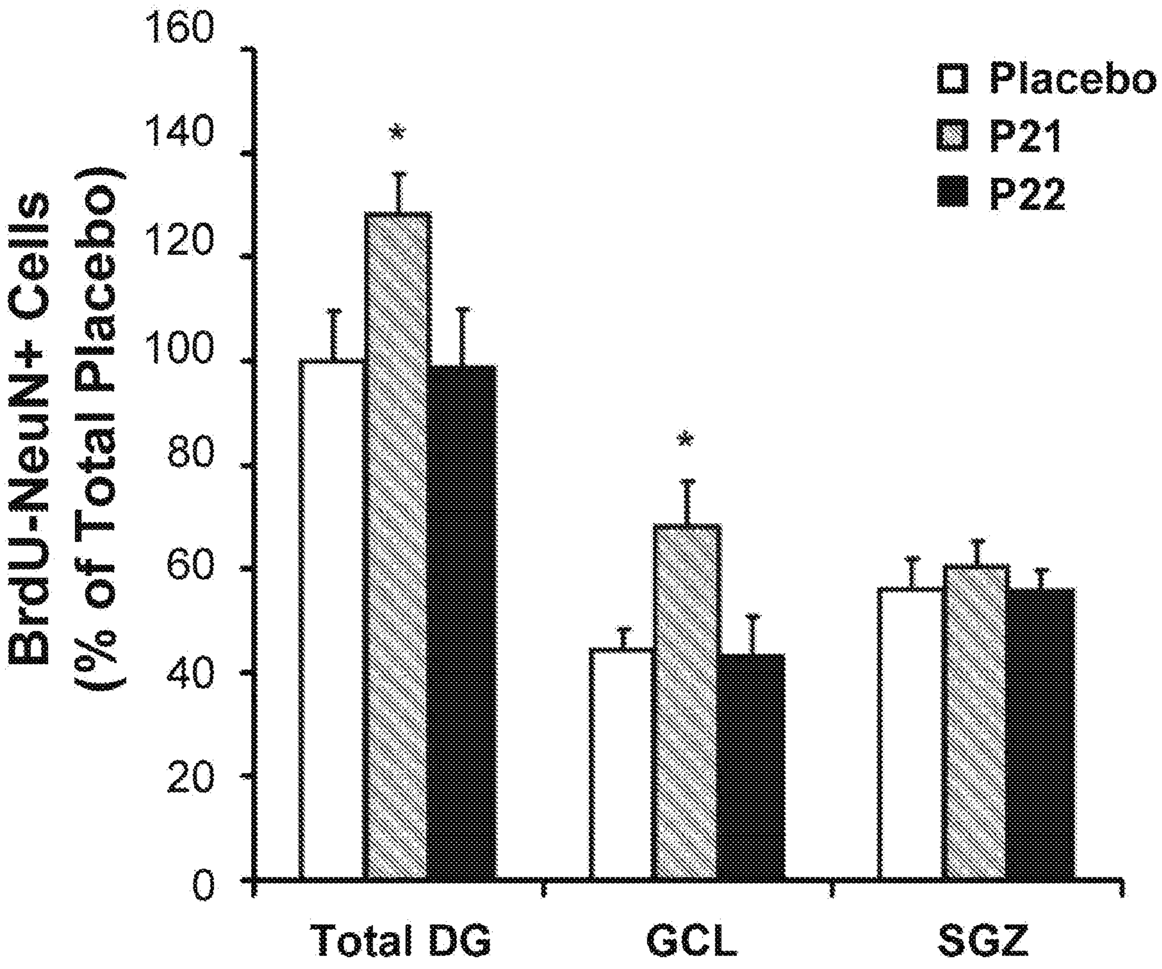

BrdU-NeuN+ Cells (% of Total Placebo)
160
140
120
100
80
60
40
20
0
Placebo
P21
P22
*
*
Total DG
GCL
SGZ

Fig. 3

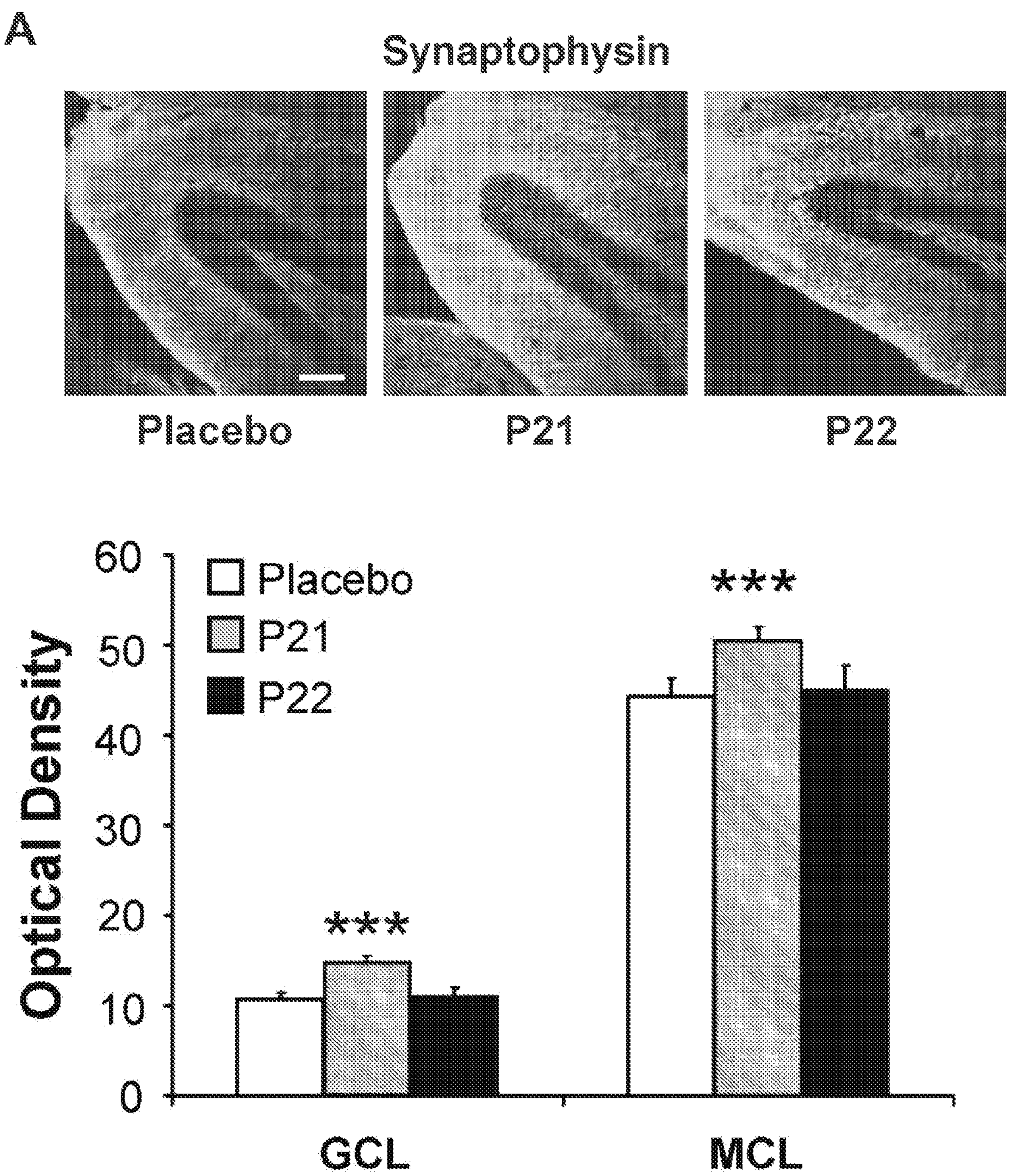
A
Synaptophysin
Placebo
P21
P22
60
50
40
30
20
10
0
Optical Density
Placebo
P21
P22
***
***
GCL
MCL

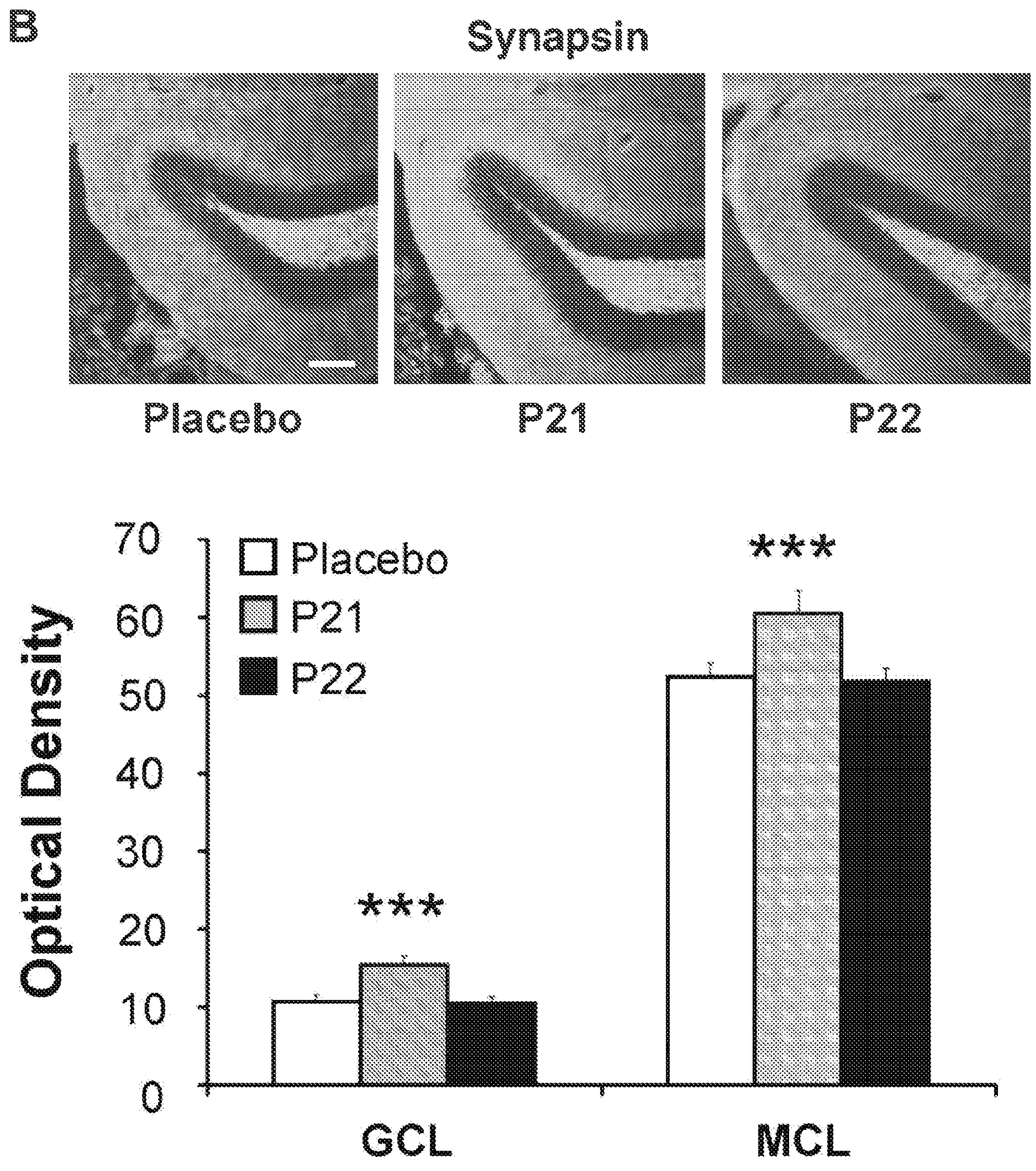
B
Synapsin
Placebo
P21
P22
70
60
50
40
30
20
10
0
Optical Density
Placebo
P21
P22
***
***
GCL
MCL

Fig. 4

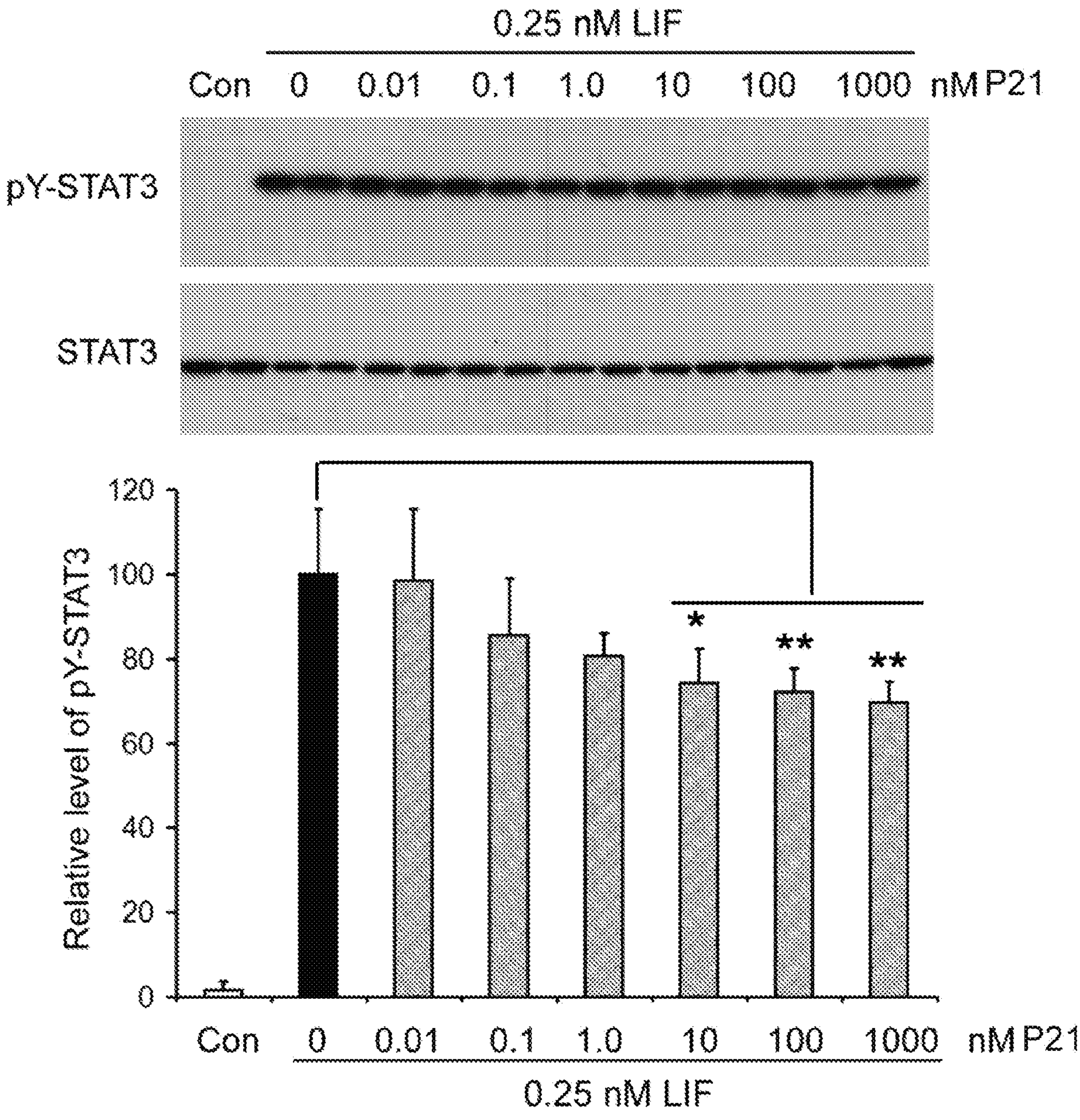
0.25 nM LIF
Con 0 0.01 0.1 1.0 10 100 1000 nM P21
pY-STAT3
STAT3
Relative level of pY-STAT3
120
100
80
60
40
20
0
*
**
**
Con 0 0.01 0.1 1.0 10 100 1000 nM P21
0.25 nM LIF

Europäisches Patentamt
European Patent Office
Office européen des brevets

# EUROPEAN SEARCH REPORT

Application Number
EP 10 19 5232

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | L. WANKAC. CABRELEM. VANEJEWSP. R. SCHREINER: "Gamma-aminoadamantanecarboxylic acids through direct C-H bond amidations" EUR. J. ORG. CHEM., vol. 2007, no. 9, 20 July 2001 (2001-07-20), pages 1474-1490, XP002619907 * compounds 45,46 * ----- | 1,2,5 | INV. C07K7/02 C07K7/56 A61P25/28 |
| X | US 4 273 704 A (MAZUR ROBERT H) 16 June 1981 (1981-06-16) * column 1, line 9 - line 10; example 20 * * column 4, lines 1-6,60-65 * ----- | 1,2,5-8 | |
| X,P | LI, BIN ET AL: "Neurotrophic peptides incorporating adamantane improve learning and memory, promote neurogenesis and synaptic plasticity in mice" FEBS LETTERS, vol. 584, no. 15, 2010, pages 3359-3365, XP002619908 * the whole document * ----- | 1-9 | TECHNICAL FIELDS SEARCHED (IPC) C07K |
| A | WO 2009/026422 A2 (ABBOTT LAB [US]; BITNER R SCOTT [US]; BROWMAN KAITLIN E [US]; BRUNE MI) 26 February 2009 (2009-02-26) * paragraph [0002] * formula (I) on page 14 * paragraph [0080] - paragraph [0287] * ----- | 3,4,8,9 | |

The present search report has been drawn up for all claims

| Place of search | Date of completion of the search | Examiner |
|---|---|---|
| The Hague | 2 February 2011 | Fuhr, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

## ANNEX TO THE EUROPEAN SEARCH REPORT ON EUROPEAN PATENT APPLICATION NO. EP 10 19 5232

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2011

| Patent document cited in search report | Publication date | Patent family member(s) | Publication date |
| --- | --- | --- | --- |
| US 4273704 A | 16-06-1981 | AU 6499080 A | 11-06-1981 |
| | | BE 886472 A1 | 03-06-1981 |
| | | CA 1151642 A1 | 09-08-1983 |
| | | DE 3044793 A1 | 19-06-1981 |
| | | DK 513480 A | 09-07-1981 |
| | | ES 8205753 A1 | 01-11-1982 |
| | | ES 8300087 A1 | 01-01-1983 |
| | | FR 2476073 A1 | 21-08-1981 |
| | | GB 2066262 A | 08-07-1981 |
| | | IT 1127930 B | 28-05-1986 |
| | | JP 56092847 A | 27-07-1981 |
| | | NL 8006586 A | 01-07-1981 |
| | | NO 803640 A | 04-06-1981 |
| | | PT 72141 A | 01-01-1981 |
| | | SE 8008428 A | 04-06-1981 |
| | | ZA 8007513 A | 28-07-1982 |
| WO 2009026422 A2 | 26-02-2009 | JP 2009046464 A | 05-03-2009 |
| | | US 2009054426 A1 | 26-02-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 197999765 A, Mazur
- WO 2006010362 A1, Schreiner
- WO 2006097774 A1


### Non-patent literature cited in the description


- **L. WANKA ; C. CABRELE ; M. VANEJEWS ; P. R. SCHREINER.** *Eur. J. Org. Chem.,* 2007, 1474
- **C. E. MÜLLER ; L. WANKA ; K. JEWELL ; P. R. SCHREINER.** *Angew. Chem. Int. Ed. Engl.,* 2008, vol. 47, 6180
- **C. E. MÜLLER ; D. ZELL ; P. R. SCHREINER.** *Chem. Eur. J.,* 2009, vol. 15, 9647
- **S. HORVAT ; K. MLINARIC-MAJERSKI ; L. GLAVAS-OBROVAC ; A. JAKAS ; J. VELJKOVIC ; S. MARCZI ; G. KRAGOL ; M. ROSCIC ; M. MATKOVIC ; A. MILOSTIC-SRB.** *J. Med. Chem.,* 2006, vol. 49, 3136
- **P. D. O'LEARY ; R. A. HUGHES.** *J. Neurochem.,* 1998, vol. 70, 1712
- **P. D. O'LEARY ; R. A. HUGHES.** *J. Biol. Chem.,* 2003, vol. 278, 25738
- **J. M. FLETCHER ; R. A. HUGHES.** *J. Pept. Sci.,* 2006, vol. 12, 515
- **J. M. FLETCHER ; C. J. MORTON ; R. A. ZWAR ; S. S. MURRAY ; P. D. O'LEARY ; R. A. HUGHES.** *J. Biol. Chem.,* 2008, vol. 283, 33375
- **J. M. FLETCHER ; R. A. HUGHES.** *Bioorg. Med. Chem.,* 2009, vol. 17, 2695
- **H. H. CHEN ; Y. C. TUNG ; B. LI ; K. IQBAL ; I. GRUNDKE-IQBAL.** *Neurobiol. Aging,* 2007, vol. 28, 1148
- **Y. MATSUOKA ; Y. JOUROUKHIN ; A. J. GRAY ; L. MA ; C. HIRATA-FUKAE ; H. F. LI ; L. FENG ; L. LECANU ; B. R. WALKER ; E. PLANEL.** *J Pharmacol Exp Ther,* 2008, vol. 325, 146